# EUROPÄISCHE PATENTANMELDUNG

(11) **EP 1 279 673 A1**
(43) Veröffentlichungstag der Anmeldung: **29.01.2003**
(21) Anmeldenummer: 02016485.1
(22) Anmeldetag: 23.07.2002
(51) Int. Cl.: C07D 403/04, C07D 403/14

(54) **Imidotriazinderivate**

(30) Priorität: 26.07.2001 AT 11752001
(71) Anmelder: Agrolinz Melamin GmbH, A-4021 Linz (AT)
(72) Erfinder: Rätzsch, Manfred, Prof. Dr., 4062 Wilhering/Thalheim (AT); Rametsteiner, Reinhard, Dipl.-Ing., 4030 Linz (AT); Schmidt, Harald, Prof. Dr., 4040 Linz (AT); Burger, Martin, Dr., 4020 Linz (AT)
(74) Vertreter: Gross, Felix

(57) **Zusammenfassung**

Alkenyl-substituierte Imidotriazinderivate der Formel (I) 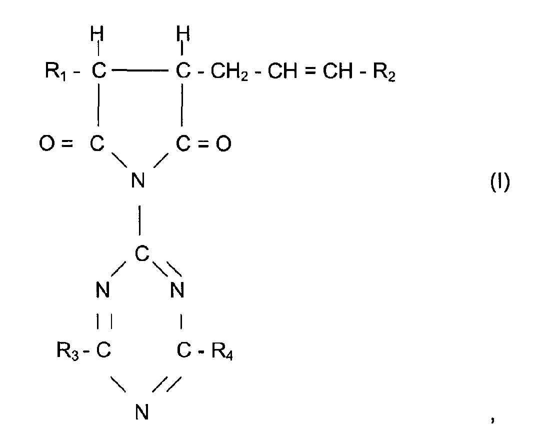 und/oder der Formel (II) 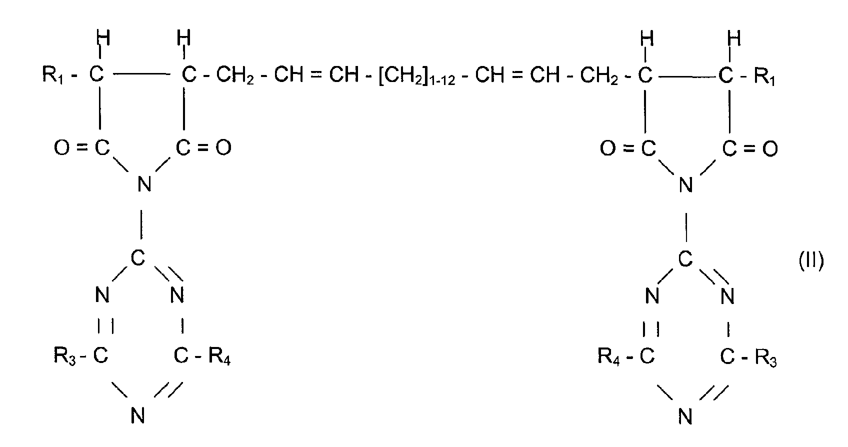 in der
R₁ = H, -CH₃, -C₂H₅,
R₂ = H, C₁-C₁₅-Alkyl, C₁-C₁₅-Alkyloxa, Cyclopentyl, Cyclohexyl, C₆-C₁₂-Aryl, C₆-C₁₂-Aryloxa, Cyano, Carboxy, - [CH₂-]₁₋₁₃ - CH = CH₂,
R₃ = -NH₂, -NH-CHR₅-OR₅ oder -N[CHR₅-O-R₅]_{2,}
R₄ = R₃, -CH₃, -C₃H₇, -C₆H₅;
R₅ = H, C₁-C₇-Alkyl,
bedeuten, deren Herstellung und deren Verwendung.

## Beschreibung

Die Erfindung betrifft substituierte Imidotriazinderivate sowie ein Verfahren zu deren Herstellung.

Gesättigte polyfunktionelle cyclische Imidotriazine wie 2,4,6-Tris-(succinimido)-1,3,5-triazin, 2,4-Bis-(succinimido)-6-amino-1,3,5-triazin, 2,4,6-Tris(succinimido)-1,3,5-triazin oder Bis-(hexahydrophthalimido)-1,3,5-triazin sind bekannt (WO 97 31 914).

Weiterhin bekannt sind Imidotriazinderivate mit ungesättigten Gruppen in Form von Imiden aus Dien/Maleinsäureanhydrid- Diels-Alder-Addukten und Melamin wie N-Mono-tetrahydrophthaloylmelamin oder N-Mono(4-methyl)tetrahydrophthaloylmelamin (WO 94 13 664). Von Nachteil bei diesen mit ethylenisch ungesättigten kondensierten Ringverbindungen substituierten Melaminderivaten ist die geringe Reaktivität der Doppelbindung und die sperrige Struktur des kondensierten Ringsystems, die insbesondere bei einer Verwendung der Imidotriazinderivate bei Polymersynthesen zu Produkten unbefriedigender Flexibilität führt.

Aufgabe der Erfindung sind Imidotriazinderivate mit ethylenisch ungesättigten Gruppen, die nicht Bestandteil eines Ringsystems sind.

Die erfinderische Aufgabe wurde durch Alkenyl-substituierte Imidotriazinderivate gelöst, wobei die Alkenyl-substituierten Imidotriazinderivate Imidotriazinderivate der Formel (I) 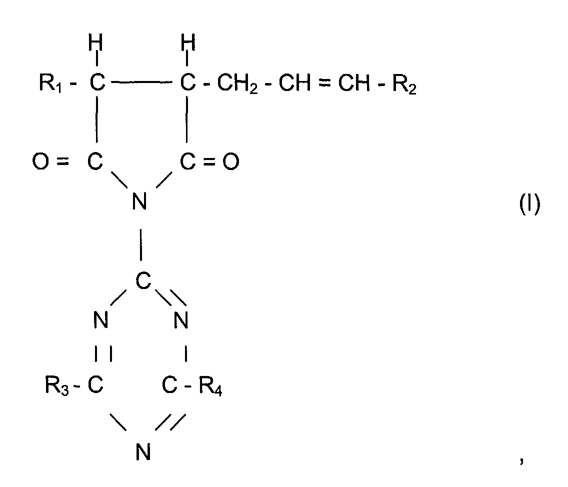 und/oder Imidotriazinderivate der Formel (II) 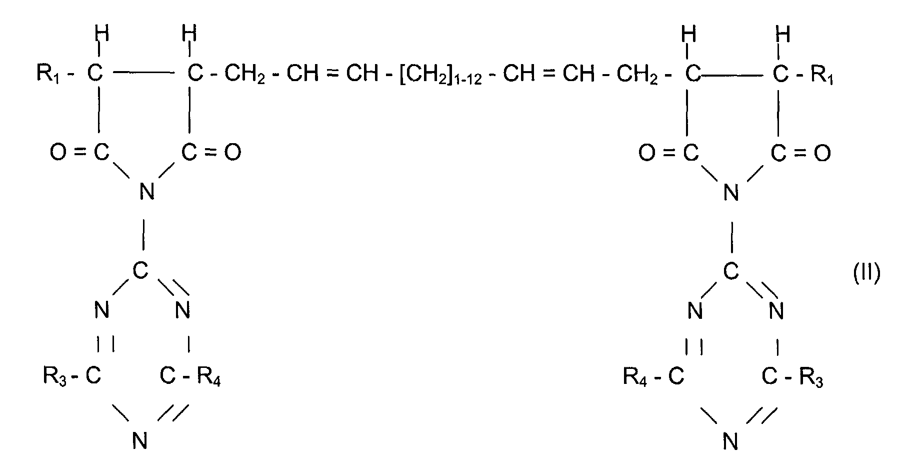 sind, wobei
- R₁ =: H, -CH₃, -C₂H₅,
- R₂ =: H, C₁-C₁₅-Alkyl, C₁-C₁₅-Alkyloxa, Cyclopentyl, Cyclohexyl, C₆-C₁₂-Aryl, C₆-C₁₂-Aryloxa, Cyano, Carboxy, - [CH₂-]₁₋₁₃ - CH = CH₂,
- R₃ =: -NH₂, -NH-CHR₅-OR₅ oder -N[CHR₅-O-R₅]₂,
- R₄ =: R₃, -CH₃, -C₃H₇, -C₆H₅;
- R₅ =: H, C₁-C₇-Alkyl,
bedeuten.

Bevorzugt werden Imidotriazinderivate, bei denen in der Formel (I) der Substituent R₁ = H ist. Beispiele für diese Imidotriazinderivate sind 2-Butenyl-2-succinimido-4,6-diamino-1,3,5-triazin, 2-Dodecenyl-2-succinimido-4-methyl-6-methylolamino-1,3,5-triazin oder 2-Octenyl-2-succinimido-4,6-di(methyloxamethylamino)-1,3,5-triazin.

Weiterhin bevorzugt sind Imidotriazinderivate der allgemeinen Formel (I), bei denen der Substituent R₃ eine Aminogruppe ist. Beispiele für diese Imidotriazinderivate sind 2-Butenyl-3-methyl-2-succinimido-4,6-diamino-1,3,5-triazin, 2-Dodecenyl-2-succinimido-4-methyl-6-amino-1,3,5-triazin oder 2-Octenyl-3-ethyl-2-succinimido-4,6-diamino-1,3,5-triazin.

Bevorzugt sind ebenfalls Imidotriazinderivate der Formel (I), bei denen der Substituent R₄ eine Aminogruppe ist. Beispiele für diese Imidotriazinderivate sind 2-Butenyl-2-succin-imido-4,6-diamino-1,3,5-triazin, 2-Dodecenyl-2-succinimido-4,6-diamino-1,3,5-triazin oder 2-Hexenyl-2-succinimido-4,6-diamino-1,3,5-triazin.

Beispiele für Imidotriazinderivate der Formel (II) sind Bis(2-propenyl-2-succinimido-4,6-diamino-1,3,5-triazin)methan, 1,2-Bis(2-butenyl-2-succinimido-4-methyl-6-amino-1,3,5- triazin)ethan oder Bis(2-propenyl-3-ethyl-2-succinimido-4,6-diamino-1,3,5-triazin)methan.

Die erfindungsgemässen Alkenyl-substituierten Imidotriazinderivate werden nach einem Mehrstufenverfahren hergestellt, wobei
- in der ersten Verfahrensstufe C₃-C₁₈-ungesättigte Verbindungen oder C₅-C₁₈-α,ω-Diolefine an ungesättigte Dicarbonsäureanhydride angelagert,
- in der zweiten Verfahrensstufe die Alkenyl- bzw. Dialkenyl-modifizierten Dicarbonsäureanhydride mit Aminotriazinderivaten umgesetzt,
- und die Alkenyl-substituierten Imidotriazinderivate gegebenenfalls in einer dritten Verfahrensstufe hydroxyalkyliert und gegebenenfalls nachfolgend verethert werden.

Imidotriazinderivate der Formel I; in der
- R₂ =: H, C₁-C₁₅-Alkyl, C₁-C₁₅-Alkyloxa, Cyclopentyl, Cyclohexyl, C₆-C₁₂-Aryl, C₆-C₁₂-Aryloxa, Cyano, Carboxyl
bedeutet, werden nach einem Mehrstufenverfahren hergestellt, wobei
in der ersten Verfahrensstufe ungesättigte Dicarbonsäureanhydride vom Typ Maleinsäureanhydrid, Itakonsäureanhydrid oder Ethylmaleinsäureanhydrid mit α-Olefinen vom Typ C₃-C₁₈-α-Olefinen, C₃-C₁₅-3-oxa-propen-1, 3-C₅-C₆-Cycloalkyl-propen-1, 3-C₆-C₁₂-Aryl-propen-1, C₆-C₁₂-Aryl-3-oxa-propen-1, 3-Cyanopropen-1 oder 3-Carboxypropen-1 im Rührreaktor bei einem Molverhältnis ungesättigtes Dicarbonsäureanhydrid / α-Olefin 1: 1 bis 1 : 1,4 in aromatischen Lösungsmitteln vom Typ Benzen, Toluen oder Xylen, wobei die Konzentration der Summe der Reaktanten im aromatischen Lösungsmittel 20 bis 70 Masse% beträgt, bei 170 bis 240°C/ 1,5 bis 20 bar und Verweilzeiten von 3 bis 12 Std. umgesetzt und nach Abdestillation des Lösungsmittels die gebildeten Alkenyl-modifizierten Dicarbonsäureanhydride durch Vacuumdestillation bei 0,005 bis 0,1 bar/160 bis 280°C abgetrennt werden,
in der zweiten Verfahrensstufe die Alkenyl-modifizierten Dicarbonsäureanhydride mit Aminotriazinderivaten vom Typ Melamin, Acetoguanamin, Butyroguanamin oder Benzoguanamin bei einem Molverhältnis Aminotriazinderivat/modifiziertes Dicarbonsäureanhydrid 1 : 1 bis 1 : 3,5 in Rührreaktoren
- entweder in polaren Lösungsmitteln vom Typ Dimethylsulfoxid, Tetramethylensulfon, Dimethylformamid oder Dimethylacetamid bzw. deren Mischungen, wobei die Konzentration der Summe der Reaktanten im Lösungsmittel bzw. Lösungsmittelgemisch 20 bis 70 Masse% beträgt, bei 80 bis 140°C und Verweilzeiten von 2,5 bis 8 Std. umgesetzt werden, und nach Abdestillation des Lösungsmittels bei 80 bis 150°C/0,01 bis 0,2 bar dem viscosen Rückstand das 3- bis 10 fache Volumen an C₁-C₄-Alkoholen zugesetzt, und die ausgefallenen Alkenyl-substituierten Imidotriazinderivate nach Abtrennung einer Kaltextraktion mit C₁-C₄-Alkoholen und Ether und gegebenenfalls einer Umkristallisation aus C₁-C₄-Alkoholen unterzogen werden,
- oder in der Schmelze bei 135 bis 210°C bei einer Verweilzeit von 45 bis 300 min umgesetzt werden, und die Alkenyl-substituierten Imidotriazinderivate nach Abkühlung auf 30 bis 65°C einer 5 bis 15 min Heissextraktion durch C₁-C₄-Alkohole und gegebenenfalls einer Umkristallisation aus C₁-C₄-Alkoholen unterzogen werden, und
gegebenenfalls in einer dritten Verfahrensstufe die Alkenyl-substituierten Imidotriazinderivate in eine Lösung von C₁-C₈-Aldehyden in Wasser eingetragen werden, wobei das Molverhältnis Alkenyl-substituiertes Imidotriazinderivat / C₁-C₈-Aldehyd 1 : 1 bis 1 : 2,5 und die Konzentration der Summe der Reaktanten in Wasser 15 bis 70 Masse% beträgt, und das Reaktionsgemisch 10 bis 90 min auf 50 bis 90°C erwärmt und die resultierenden Hydroxyalkyl-modifizierten Alkenyl-substituierten Imidotriazinderivate durch Sprühtrocknung getrocknet werden, oder die Hydroxyalkyl-modifizierten Alkenyl-substituierten Imidotriazinderivate durch Zusatz eines Überschusses an C₁-C₃-Alkoholen zur wässrigen Lösung bzw. Dispersion im sauren Medium bei 5 bis 80°C verethert und gegebenenfalls durch azeotrope Umetherung mit C₄-C₁₆-Alkoholen in die entsprechenden C₄-C₁₆-Alkylether übergeführt werden.
Beispiele für geeignete C₃-C₁₈-α-Olefine, die bei dem erfindungsgemässen Verfahren zur Herstellung von Imidotriazinverbindungen der Formel (I) in der ersten Verfahrensstufe eingesetzt werden, sind Buten-1, Octen-1 und Dodecen-1.
Beispiele für geeignete α-Olefine vom Typ C₃-C₁₅-3-oxa-propen-1, die bei dem erfindungsgemässen Verfahren zur Herstellung von Imidotriazinverbindungen der Formel (I) in der ersten Verfahrensstufe eingesetzt werden, sind n-Propyl-3-oxa-propen-1, Ethylhexyl-3-oxa-propen-1 und Dodecyl-3-oxa-propen-1.
Beispiele für geeignete α-Olefine vom Typ 3-C₅-C₆-Cycloalkyl-propen-1, die bei dem erfindungsgemässen Verfahren zur Herstellung von Imidotriazinverbindungen der Formel (I) in der ersten Verfahrensstufe eingesetzt werden, sind 3-Cyclopentylpropen-1 oder 3-Cyclo-hexylpropen-1.
Beispiele für geeignete α-Olefine vom Typ 3-C₆-C₁₂-Aryl-propen-1, die bei dem erfindungsgemässen Verfahren zur Herstellung von Imidotriazinverbindungen der Formel (I) in der ersten Verfahrensstufe eingesetzt werden, sind 3-Phenylpropen-1 oder 3-Toluyl-propen-1.
Beispiele für geeignete α-Olefine vom Typ C₆-C₁₂-Aryl-3-oxa-propen-1, die bei dem erfindungsgemässen Verfahren zur Herstellung von Imidotriazinverbindungen der Formel (I) in der ersten Verfahrensstufe eingesetzt werden, sind Phenyl-3-oxapropen-1 oder Benzyl-3-oxa-propen-1.
Geeignete C₁-C₄-Alkohole, die in der zweiten Verfahrensstufe für die Extraktion bzw. Umkristallisation der Alkenyl-substituierten Triazinderivate eingesetzt werden können, sind Methanol, Ethanol und tert. Butanol.
Geeignete C₁-C₈-Aldehyde, die in der dritten Verfahrensstufe für die Hydroxyalkylierung der Alkenyl-substituierten Triazinderivate eingesetzt werden können, sind Formaldehyd oder Glyoxal.

Imidotriazinderivate der Formel I, in der R₂ = - [Ch₂-]₁₋₁₃ - CH = CH₂ bedeutet, und Imidotriazinderivate der Formel II
werden nach einem Mehrstufenverfahren hergestellt, wobei
in der ersten Verfahrensstufe ungesättigte Dicarbonsäureanhydride vom Typ Maleinsäureanhydrid, Itakonsäureanhydrid oder Ethylmaleinsäureanhydrid mit C₇-C₁₈-α,ω-Diolefinen im Rührreaktor bei einem Molverhältnis C₇-C₁₈-α,ω-Diolefin / ungesättigtes Dicarbonsäureanhydrid / 1: 1,4 bis 1 : 3 in aromatischen Lösungsmitteln vom Typ Benzen, Toluen oder Xylen, wobei die Konzentration der Summe der Reaktanten im aromatischen Lösungsmittel 20 bis 70 Masse% beträgt, bei 170 bis 260°C/ 1,5 bis 20 bar und Verweilzeiten von 3 bis 12 Std. umgesetzt und nach Abdestillation des Lösungsmittels die gebildeten Dialkenyl-modifizierten Dicarbonsäureanhydride durch Vacuumdestillation bei 0,001 bis 0,1 bar/160 bis 280°C, gegebenenfalls unter Fraktionierung, abgetrennt werden,
in der zweiten Verfahrensstufe die Dialkenyl-modifizierten Dicarbonsäureanhydride mit Aminotriazinderivaten vom Typ Melamin, Acetoguanamin, Butyroguanamin oder Benzoguanamin bei einem Molverhältnis Aminotriazinderivat/modifiziertes Dicarbonsäureanhydrid 1 : 1 bis 6 : 1 in Rührreaktoren
- entweder in polaren Lösungsmitteln vom Typ Dimethylsulfoxid, Tetramethylensulfon, Dimethylformamid oder Dimethylacetamid bzw. deren Mischungen, wobei die Konzentration der Summe der Reaktanten im Lösungsmittel bzw. Lösungsmittelgemisch 20 bis 70 Masse% beträgt, bei 80 bis 140°C und Verweilzeiten von 2,5 bis 8 Std. umgesetzt werden, und nach Abdestillation des Lösungsmittels bei 80 bis 150°C/0,01 bis 0,2 bar dem viscosen Rückstand das 3- bis 10 fache Volumen an C₁-C₄-Alkoholen zugesetzt, und die ausgefallenen Dialkenyl-substituierten Imidotriazinderivate nach Abtrennung einer Kaltextraktion mit C₁-C₄-Alkoholen und Ether und gegebenenfalls einer Umkristallisation aus C₁-C₄-Alkoholen unterzogen werden,
- oder in der Schmelze bei 135 bis 210°C bei einer Verweilzeit von 45 bis 300 min umgesetzt werden, und die Dialkenyl-substituierten Imidotriazinderivate nach Abkühlung auf 30 bis 65°C einer 5 bis 15 min Heissextraktion durch C₁-C₄-Alkohole und gegebenenfalls einer Umkristallisation aus C₁-C₄-Alkoholen unterzogen werden, und
gegebenenfalls in einer dritten Verfahrensstufe die Dialkenyl-substituierten Imidotriazinderivate in eine Lösung von C₁-C₈-Aldehyden in Wasser eingetragen werden, wobei das Molverhältnis Dialkenyl-substituiertes Imidotriazinderivat / C₁-C₈-Aldehyd 1 : 1 bis 1 : 10 und die Konzentration der Summe der Reaktanten in Wasser 15 bis 70 Masse% beträgt, und das Reaktionsgemisch 10 bis 90 min auf 50 bis 90°C erwärmt und die resultierenden Hydroxyalkyl-modifizierten Dialkenyl-substituierten Imidotriazinderivate durch Sprühtrocknung getrocknet werden, oder die Hydroxyalkyl-modifizierten Dialkenyl-substituierten Imidotriazinderivate durch Zusatz eines Überschusses an C₁-C₃-Alkoholen zur wässrigen Lösung bzw. Dispersion im sauren Medium bei 5 bis 80°C verethert und gegebenenfalls durch azeotrope Umetherung mit C₄-C₁₆-Alkoholen in die entsprechenden C₄-C₁₆-Alkylether übergeführt werden.
Geeignete C₇-C₁₈-α,ω-Diolefine, die bei der Herstellung von Imidotriazinderivaten der Formel (II) und (III) in der ersten Verfahrensstufe eingesetzt werden können, sind α,ω-Heptadien, α,ω-Dodecadien und α,ω-Octadecadien.
Bevorzugt wird bei den Herstellungsverfahren von Imidotriazinderivaten der Formel (I) bis (III) in der ersten Verfahrensstufe als ungesättigtes Dicarbonsäureanhydrid Maleinsäureanhydrid eingesetzt.
Bevorzugte Reaktionsbedingungen in der ersten Verfahrensstufe sind Reaktionstemperaturen von 190 bis 220°C und Reaktionszeiten von 5 bis 8 Stunden.
In der zweiten Verfahrensstufe bei der Herstellung von Imidotriazinderivaten der Formel (I) bis (III) wird als Aminotriazinderivat insbesondere Melamin eingesetzt.

Bevorzugte Reaktionsbedingungen für die Reaktion der Alkenyl- bzw. Dialkenyl-modifizierten Dicarbonsäureanhydride mit Aminotriazinderivaten in der zweiten Verfahrensstufe bei Umsetzung im Rührreaktor in Lösung sind Reaktionstemperaturen von 100 bis 120°C und Reaktionszeiten von 4 bis 6 Stunden. Wird die Umsetzung in der zweiten Verfahrensstufe in der Schmelze durchgeführt, so werden Reaktionstemperaturen von 150 bis 180°C und Reaktionszeiten von 60 bis 180 min bevorzugt.

Die gegebenenfalls in einer dritten Verfahrensstufe durchgeführte Hydroxyalkylierung der Alkenyl- bzw. Dialkenyl-substituierten Imidotriazinderivate mit C₁- bis C₈-Aldehyden wird bevorzugt im sauren oder schwach alkalischen Medium bei pH=4,5 bis 7,5, Reaktionstemperaturen von 80 bis 85°C und Reaktionszeiten von 30 bis 50 min durchgeführt, dabei wird als Aldehyd insbesondere Formaldehyd eingesetzt.

Die gegebenenfalls weiterhin in einer dritten Verfahrensstufe durchgeführte Veretherung der hydroxyalkylierten Alkenyl- bzw. Dialkenyl-substituierten Imidotriazinderivate kann durch direkte Veretherung mit einem Überschuss an C₁-C₃-Alkoholen in stark saurem Medium, bevorzugt bei Reaktionstemperaturen von 40 bis 70°C und Reaktionszeiten von 40 bis 90 min, und nachfolgender Abdestillation des Azeotrops aus nicht umgesetztem Alkohol und Wasser, durchgeführt werden. Die Herstellung von C₄- bis C₁₆-Ethern von Imidotriazinderivaten mit Hydroxyalkyl- und Alkenyl- bzw. Dialkenyl-Gruppen erfolgt durch Umetherung der entsprechenden C₁- bis C₃-Ether mit C₄- bis C₁₆-Alkoholen unter Abdestillation der abgespaltenen C₁-C₃-Alkohole bei Reaktionstemperaturen von bevorzugt 70 bis 95°C.

Die erfindungsgemässen Imidotriazinderivate der Formel (I) und (II) sind insbesondere zur Herstellung von Polymeren, Pharmaka, Biociden, Additiven, Textilhilfsmitteln und Farbstoffen geeignet.

### Beispiel 1

### 1.1 Herstellung des Alkenyl-modifizierten Dicarbonsäureanhydrids

In einen 5 I - Rührautoklav werden 0,5 kg Maleinsäureanhydrid, 1 kg Dodecen-1 und 1 kg Benzen dosiert. Nach Erwärmung der Reaktionsmischung auf 190oC und Homogenisierung wird 7 Std. bei 210oC/19 bar umgesetzt, das Gemisch aus Benzen und nicht-umgesetztem Dodecen-1 nach Entspannung abdestilliert und nachfolgend das 2-Dodecenyl-bernsteinsäureanhydrid bei 210-220oC/ 0,05 bar abdestilliert.

### 1.2 Herstellung des Alkenyl-substituierten Imidotriazinderivats

In einen 6 I Rührreaktor werden 126 g Melamin, 532 g des Alkenyl-modifizierten Dicarbon-säureanhydrids nach 1.1 und 3 I einer Mischung aus Dimethylsulfoxid/Dimethylacetamid 2:1 dosiert. Nach Erwärmung auf 115°C wird 5 Std. gerührt und das Lösungsmittelgemisch bei 135°C/0.1 bar abdestilliert. Nach Zusatz von 3 I Ethanol bildet sich eine leicht gelblich gefärbte Dispersion, die durch das Bodenventil ausgetragen, abfiltriert und mit Ethanol und Ether gewaschen wird. Nach Umkristallisation aus Ethanol werden farblose Kristalle mit einem Schmelzbereich von 190 bis 205°C erhalten.

Die 1H-NMR-Analyse des 2-Dodecenyl-succinimido-4,6-diamino-1,3,5-triazins in Dimethylsulfoxid-Lösung führt zu folgendem Ergebnis:

| | | | |
|---|---|---|---|
| 1. Signal | 0.8 bis 3.3 ppm | H | -Alkyl-Kette und -CO-CH₂-CH-CO- -Gruppe |
| 2. Signal | 5.3 bis 5.5 ppm | 2H | -CH=CH- -Gruppe |
| 3. Signal | 7.11 ppm | 4H | 2 NH₂ -Gruppen |

### Beispiel 2

### 2.1 Herstellung des Alkenyl-modifizierten Dicarbonsäureanhydrids

In einen 5 I - Rührautoklav werden 565 g Itakonsäureanhydrid, 500 g Hexen-1 und 1 kg Toluen dosiert. Nach Erwärmung der Reaktionsmischung auf 190°C und Homogenisierung wird 8 Std. bei 205°C/19 bar umgesetzt, das Gemisch aus Toluen und nicht-umgesetztem Hexen-1 nach Entspannung abdestilliert und nachfolgend das Hexen-ltakonsäureanhydrid-Addukt bei 200-210oC/ 0,05 bar abdestilliert.

### 2.2 Herstellung des Alkenyl-substituierten Imidotriazinderivats

In einen 4 I Rührreaktor werden 125 g Acetoguanamin und 500 g des Alkenyl-modifizierten Dicarbonsäureanhydrids nach 2.1 dosiert, aufgeschmolzen und 120 min auf 160°C erwärmt. Nach Abkühlung auf 50°C werden 2,5 I eines Ethanollsopropanol-Gemischs 1 : 1 zugesetzt, 15 min am Rückfluss erhitzt, die leicht gelbliche Dispersion durch das Boden-ventil ausgetragen und abfiltriert. Nach Umkristallisation aus Ethanol werden farblose Kristalle mit einem Schmelzbereich von 185 bis 200°C erhalten.

### 2.3 Hydroxyalkylierung des Alkenyl-substituierten Imidotriazinderivats

In einem 2 I Rührreaktor werden 330 g des Alkenyl-substituierten Imidotriazinderivats nach 2.2 in 800 ml 10% Formalinlösung dispergiert, der pH-Wert mit NaOH auf pH=7 eingestellt, die Reaktionsmischung 90 min bei 85°C umgesetzt und durch Sprühtrocknung getrocknet.

Die IR-Analyse ergibt ein Molverhältnis Hydroxymethylgruppen/Triazinring von 2:1.

### Beispiel 3

### 3.1 Herstellung des Dialkenyl-modifizierten Dicarbonsäureanhydrids

In einen 5 I - Rührautoklav werden 0,5 kg Maleinsäureanhydrid, 0,4 kg α,ω-Octadien und 1,5 kg Benzen dosiert. Nach Erwärmung der Reaktionsmischung auf 195°C und Homogenisierung wird 8 Std. bei 210°C/19 bar umgesetzt, das Gemisch aus Benzen und nicht-umgesetztem α,ω-Octadien nach Entspannung abdestilliert, die Schmelze in einen Dünnschichtverdampfer übergeführt und nachfolgend das α,ω-Octadien-Maleinsäureanhydrid-Addukt fraktioniert bei 0,05 bar abdestilliert. Die Ausbeute der Fraktion 1 bei 190-235°C/0,05 bar beträgt 235 g, die Ausbeute der Fraktion 2 bei 235-280°C/0,05 bar beträgt 160 g.
- Fraktion 1: 2-( Octadienyl-2,7 )-bernsteinsäureanhydrid
- Fraktion 2: 1, 8-Bis- (2-succinyl)-octadien-2, 6

IR-Daten (KBr, cm-1):
   2956, 2925, 2854 -CH-
   1864,1785 C=O
1H-NMR (200 MHz, DMSO):
   0,80 - 3,28 m, 14 H
   5,25 - 5,52 m, 4 H

### 3.2 Herstellung der Dialkenyl-substituierten Imidotriazinderivate

In einen 3 I Rührreaktor werden 70 g Melamin, 200 g der Fraktion 1 des Dialkenyl-modifizierten Dicarbonsäureanhydrids nach 3.1 und 1,4 I einer Mischung aus Dimethylsulfoxld/Dimethylacetamid 2:1 dosiert. Nach Erwärmung auf 120°C wird 6 Std. gerührt und das Lösungsmittelgemisch bei 135°C/0.1 bar abdestilliert. Nach Zusatz von 1,4 I Ethanol bildet sich eine leicht gelblich gefärbte Dispersion, die durch das Bodenventil ausgetragen, abfiltriert und mit Ethanol und Ether gewaschen wird. Nach Umkristallisation aus Ethanol werden farblose Kristalle von 2-(Octadienyl-2, 7)-succinimido-2, 4-diamino-1, 3, 5-triazin mit einem Schmelzbereich von 185 bis 215oC erhalten.
IR: 3451, 3339, 3132 NH
   2956, 2382, 285 CH
   1790, 1723, C=O
1H-NMR :
   0,83 - 3,05 m, 14 H
   5,25 - 5,53 m, 4 H
   7,11, s 4 H

Für die Umsetzung der Fraktion 2 des Dialkenyl-modifizierten Dicarbonsäureanhydrids zum Imidotriazinderivat werden in einen 3 I Rührreaktor 80 g Melamin, 160 g der Fraktion 2 des Dialkenyl-modifizierten Dicarbonsäureanhydrids nach 3.1 und 1,6 I einer Mischung aus Dlmethylsulfoxld/Dlmethylacetamid 2:1 dosiert. Nach Erwärmung auf 130°C wird 7 Std. gerührt und das Lösungsmittelgemisch bei 135°C/0.1 bar abdestilliert. Nach Zusatz von 1,6 I Ethanol bildet sich eine gelblich gefärbte Dispersion, die durch das Bodenventil ausgetragen, abfiltriert und mit Ethanol und Ether gewaschen wird. Nach Umkristallisation aus Ethanol werden farblose Kristalle von 1, 8-Bis- (2-succinimido-2, 4-diamino-1, 3, 5-triazin)-octadien-2, 6 mit einem Schmelzbereich von 235 bis 255°C erhalten.
IR: 3427, 3326, 3132 NH
   2958, 2926, 2854 CH
1H-NMR : 0,8 - 3,02 m, 14 H
   5,29 - 5,56 m, 4 H
   7,06, s 8 H

### Beispiel 4

### 4.1 Herstellung des Alkenyl-modifizierten Dicarbonsäureanhydrids

In einen 5 I - Rührautoklav werden 630 g Ethylmaleinsäureanhydrid, 350 g Buten-1 und 1 kg Toluen dosiert. Nach Erwärmung der Reaktionsmischung auf 190oC und Homogenisierung wird 7 Std. bei 205oC/20 bar umgesetzt, das Gemisch aus Toluen und nicht-umgesetztem Buten-1 nach Entspannung abdestilliert und nachfolgend das 2-Butenyl-bernsteinsäureanhydrid bei 200-210 °C/ 0,05 bar abdestilliert.

### 4.2 Herstellung des Alkenyl-substituierten Imidotriazinderivats

In einen 4 I Rührreaktor werden 126 g Melamin und 370 g des Alkenyl-modifizierten Dicarbonsäureanhydrids nach 4.1 dosiert, aufgeschmolzen und 100 min auf 160oC erwärmt. Nach Abkühlung auf 50oC werden 2,5 I Ethanol zugesetzt, 15 min am Rückfluss erhitzt, die leicht gelbliche Dispersion durch das Bodenventil ausgetragen und abfiltriert. Nach Umkristallisation aus Ethanol werden farblose Kristalle von 2-Butenyl-succinimido-2, 4-diamino-1, 3, 5-triazin mit einem Schmelzbereich von 180 bis 200 °C erhalten.

### 4.3 Hydroxyalkylierung und Veretherung des Alkenyl-substituierten Imidotriazinderivats

In einem 2 I Rührreaktor werden 290 g des Alkenyl-substituierten Imidotriazinderivats nach 4.2 in 650 ml 10% Formalinlösung dispergiert, der pH-Wert mit ethanolischer NaOH auf pH=7 eingestellt, die Reaktionsmischung 60 min bei 85°C umgesetzt und durch Sprühtrocknung getrocknet.
Die IR-Analyse ergibt ein Molverhältnis Hydroxymethyigruppen/Triazinring von 2 : 1.

Zur Veretherung werden in einem 2 I - Rührreaktor 200 g des methylolierten Imidotriazinderivats nach 4.3 in eine Mischung aus 1 I Methanol und 5 ml konzentrierter Salzsäure innerhalb 1 Std. eingetragen, 1 Std. bei 50°C gerührt und unter Abdestillation des nicht-umgesetzten Methanols 30 min auf 85°C erwärmt. Nach Zugabe von 100 ml Ethylhexanol wird unter Abdestillation des abgespaltenen Methanols 1,5 Std. auf 85°C erwärmt, und das nichtumgesetzte Ethylhexanol bei 105°C/ 0,05 bar abdestilliert.
Nach Umkristallisation aus Ethanol werden farblose Kristalle von 2-Butenylsuccinimido-2, 4-dimethoxydiamino-1, 3, 5-triazin mit einem Schmelzbereich von 175 bis 195 °C erhalten.

Analog wurden folgende Verbindungen hergestellt:
2-Cyclohexenyl-succinimido-2, 4-diamino-1, 3, 5-triazin
2-Decenyl-succinimido-2,4-diamino-1, 3, 5- triazin
2-Tetradeceyl-succinimido-2, 4-diamino-1, 3, 5, -triazin

## Patentansprüche

1. Imidotriazinderivate, **dadurch gekennzeichnet, dass** die Imidotriazinderivate Alkenyl-substituierte Imidotriazinderivate der Formel (I) 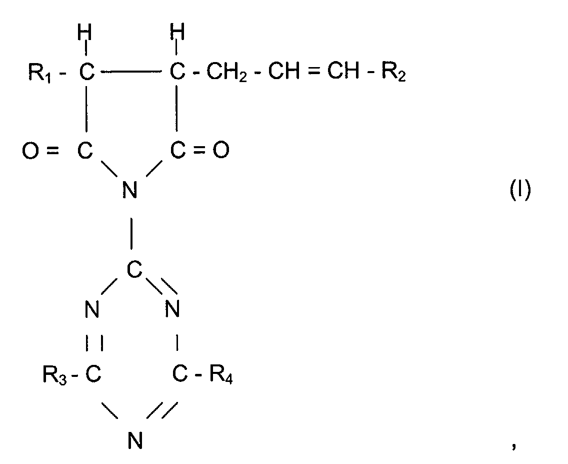 und/oder der Formel (II) 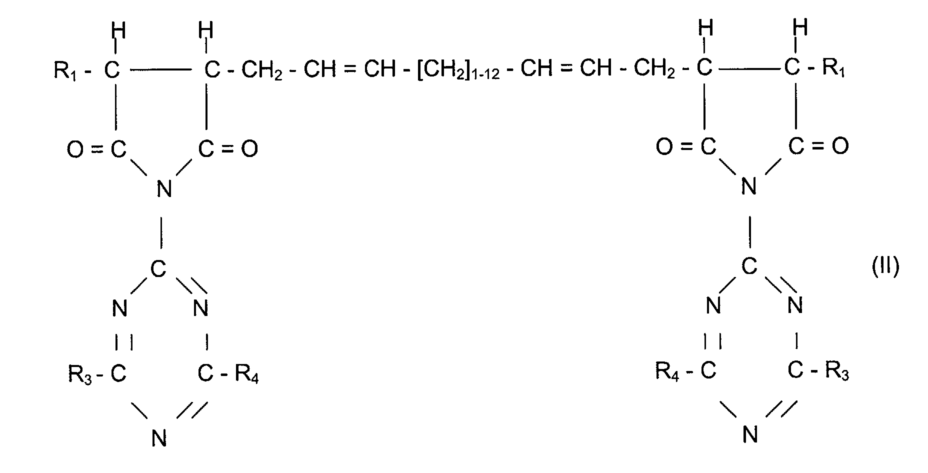 sind, wobei
R₁ = H, -CH₃, -C₂H₅,
R₂ = H, C₁-C₁₅-Alkyl, C₁-C₁₅-Alkyloxa, Cyclopentyl, Cyclohexyl, C₆-C₁₂-Aryl, C₆-C₁₂- Aryloxa, Cyano, Carboxy, - [CH₂-]₁₋₁₃ - CH = CH₂,
R₃ = -NH₂, -NH-CHR₅-OR₅ oder -N[CHR₅-O-R₅]₂,
R₄ = R₃, -CH₃, -C₃H₇, -C₆H₅;
R₅ = H, C₁-C₇-Alkyl,
bedeuten.

2. Imidotriazinderivate nach Anspruch 1, **dadurch gekennzeichnet, dass** in den Imidotriazinderivaten der Formel (I) der Substituent R₁ = H ist.

3. Imidotriazinderivate nach Anspruch 1 oder 2, **dadurch gekennzeichnet, dass** in den Imidotriazinderivaten der Formel (I) der Substituent R₃ eine Aminogruppe ist.

4. Imidotriazinderivate nach einem oder mehreren der Ansprüche 1 bis 4 , **dadurch gekennzeichnet, dass** in den Imidotriazinderivaten der Formel (I) der Substituent R₄ eine Aminogruppe ist.

5. Verfahren zur Herstellung von Imidotriazinderivaten der Formel I nach Anspruch 1, in der
R₁ = H, CH₃, C₂H₅,
R₂ = H, C₁-C₁₅-Alkyl, C₁-C₁₅-Alkyloxa, Cyclopentyl, Cyclohexyl, C₆-C₁₂-Aryl, C₆-C₁₂-Aryloxa, Cyano, Carboxyl,
R₃ = -NH₂, -NH-CHR₅-OR₅ oder -N[CHR₅-O-R₅]₂,
R₄ = R₃, -CH₃, -C₃H₇, -C₆H₅;
R₅ = H, C₁-C₇-Alkyl
bedeuten, **dadurch gekennzeichnet, dass**
in der ersten Verfahrensstufe ungesättigte Dicarbonsäureanhydride vom Typ Maleinsäureanhydrid, Itakonsäureanhydrid oder Ethylmaleinsäureanhydrid mit α-Olefinen vom Typ C₃-C₁₈-α-Olefinen, C₃-C₁₅-3-oxa-propen-1, 3-C₅-C₆-Cycloalkyl-propen-1, 3-C₆-C₁₂-Aryl-propen-1, C₆-C₁₂-Aryl-3-oxa-propen-1, 3-Cyanopropen-1 oder 3-Carboxypropen-1 im Rührreaktor bei einem Molverhältnis ungesättigtes Dicarbonsäureanhydrid / α-Olefin 1: 1 bis 1 : 1,4 in aromatischen Lösungsmitteln vom Typ Benzen, Toluen oder Xylen, wobei die Konzentration der Summe der Reaktanten im aromatischen Lösungsmittel 20 bis 70 Masse% beträgt, bei 170 bis 240°C/ 1,5 bis 20 bar und Verweilzeiten von 3 bis 12 Std. umgesetzt und nach Abdestillation des Lösungsmittels die gebildeten Alkenyl-modifizierten Dicarbonsäureanhydride durch Vacuumdestillation bei 0,005 bis 0,1 bar/160 bis 280°C abgetrennt werden,
in der zweiten Verfahrensstufe die Alkenyl-modifizierten Dicarbonsäureanhydride mit Aminotriazinderivaten vom Typ Melamin, Acetoguanamin, Butyroguanamin oder Benzoguanamin bei einem Molverhältnis Aminotriazinderivat/modifiziertes Dicarbonsäureanhydrid 1 : 1 bis 1 : 3,5 in Rührreaktoren
- entweder in polaren Lösungsmitteln vom Typ Dimethylsulfoxid, Tetramethylensulfon, Dimethylformamid oder Dimethylacetamid bzw. deren Mischungen, wobei die Konzentration der Summe der Reaktanten im Lösungsmittel bzw. Lösungsmittelgemisch 20 bis 70 Masse% beträgt, bei 80 bis 140°C und Verweilzeiten von 2,5 bis 8 Std. umgesetzt werden, und nach Abdestillation des Lösungsmittels bei 80 bis 150°C/0,01 bis 0,2 bar dem viscosen Rückstand das 3- bis 10 fache Volumen an C₁-C₄-Alkoholen zugesetzt, und die ausgefallenen Alkenyl-substituierten Imidotriazinderivate nach Abtrennung einer Kaltextraktion mit C₁-C₄-Alkoholen und Ether und gegebenenfalls einer Umkristallisation aus C₁-C₄-Alkoholen unterzogen werden,
- oder in der Schmelze bei 135 bis 210°C bei einer Verweilzeit von 45 bis 300 min umgesetzt werden, und die Alkenyl-substituierten Imidotriazinderivate nach Abkühlung auf 30 bis 65°C einer 5 bis 15 min Heissextraktion durch C₁-C₄-Alkohole und gegebenenfalls einer Umkristallisation aus C₁-C₄-Alkoholen unterzogen werden, und
gegebenenfalls in einer dritten Verfahrensstufe die Alkenyl-substituierten Imidotriazinderivate in eine Lösung von C₁-C₈-Aldehyden in Wasser eingetragen werden, wobei das Molverhältnis Alkenyl-substituiertes Imidotriazinderivat / C₁-C₈-Aldehyd 1 : 1 bis 1 : 2,5 und die Konzentration der Summe der Reaktanten in Wasser 15 bis 70 Masse% beträgt, und das Reaktionsgemisch 10 bis 90 min auf 50 bis 90°C erwärmt und die resultierenden Hydroxyalkyl-modifizierten Alkenyl-substituierten Imidotriazinderivate durch Sprühtrocknung getrocknet werden, oder die Hydroxyalkyl-modifizierten Alkenyl-substituierten Imidotriazinderivate durch Zusatz eines Überschusses an C₁-C₃-Alkoholen zur wässrigen Lösung bzw. Dispersion im sauren Medium bei 5 bis 80°C verethert und gegebenenfalls durch azeotrope Umetherung mit C₄-C₁₆-Alkoholen in die entsprechenden C₄-C₁₆-Alkyl-ether übergeführt werden.

6. Verfahren zur Herstellung von Imidotriazinderivaten nach Anspruch 1 der Formel (I), in der R₂ = - [CH₂-]₁₋₁₃ - CH = CH₂ und von Imidotriazinderivaten der Formel (II)
wobei
R₁ = H, -CH₃, -C₂H₅,
R₃ = -NH₂, -NH-CHR₅-OR₅ oder -N[CHR₅-O-R₅]₂,
R₄ = R₃, -CH₃, -C₃H₇, -C₆H₅;
R₅ = H, C₁-C₇-Alkyl,
bedeuten, **dadurch gekennzeichnet, dass**
- in der ersten Verfahrensstufe ungesättigte Dicarbonsäureanhydride vom Typ Maleinsäureanhydrid, Itakonsäureanhydrid oder Ethylmaleinsäureanhydrid mit C7-C12-α,ω-Diolefinen im Rührreaktor bei einem Molverhältnis C₇-C₁₂-α,ω-Diolefin / ungesättigtes Dicarbonsäureanhydrid 1: 1,4 bis 1 : 3 in aromatischen Lösungsmitteln vom Typ Benzen, Toluen oder Xylen, wobei die Konzentration der Summe der Reaktanten im aromatischen Lösungsmittel 20 bis 70 Masse% beträgt, bei 170 bis 260°C/1,5 bis 20 bar und Verweilzeiten von 3 bis 12 Std. umgesetzt und nach Abdestillation des Lösungsmittels die gebildeten Dialkenyl-modifizierten Dicarbonsäureanhydride durch Vacuumdestillation bei 0,001 bis 0,1 bar/160 bis 280°C, gegebenenfalls unter Fraktionierung, abgetrennt werden,
- in der zweiten Verfahrensstufe die Dialkenyl-modifizierten Dicarbonsäureanhydride mit Aminotriazinderivaten vom Typ Melamin, Acetoguanamin, Butyroguanamin oder Benzo-guanamin bei einem Molverhältnis Aminotriazinderivat/modifiziertes Dicarbonsäureanhydrid 1 : 1 bis 6 : 1 in Rührreaktoren
- entweder in polaren Lösungsmitteln vom Typ Dimethylsulfoxid, Tetramethylensulfon, Dimethylformamid oder Dimethylacetamid bzw. deren Mischungen, wobei die Konzentration der Summe der Reaktanten im Lösungsmittel bzw. Lösungsmittelgemisch 20 bis 70 Masse% beträgt, bei 80 bis 140°C und Verweilzeiten von 2,5 bis 8 Std. umgesetzt werden, und nach Abdestillation des Lösungsmittels bei 80 bis 150°C/0,01 bis 0,2 bar dem viscosen Rückstand das 3- bis 10 fache Volumen an C₁-C₄-Alkoholen zugesetzt, und die ausgefallenen Dialkenyl-substituierten Imidotriazinderivate nach Abtrennung einer Kaltextraktion mit C₁-C₄-Alkoholen und Ether und gegebenenfalls einer Umkristallisation aus C₁-C₄-Alkoholen unterzogen werden,
- oder in der Schmelze bei 135 bis 210°C bei einer Verweilzeit von 45 bis 300 min umgesetzt werden, und die Dialkenyl-substituierten Imidotriazinderivate nach Abkühlung auf 30 bis 65°C einer 5 bis 15 min Heißextraktion durch C₁-C₄-Alkohole und gegebenenfalls einer Umkristallisation aus C₁-C₄-Alkoholen unterzogen werden, und
gegebenenfalls in einer dritten Verfahrensstufe die Dialkenyl-substituierten Imidotriazinderivate in eine Lösung von C₁-C₈-Aldehyden in Wasser eingetragen werden, wobei das Molverhältnis Dialkenyl-substituiertes Imidotriazinderivat / C₁-C₈-Aldehyd 1 : 1 bis 1 : 10 und die Konzentration der Summe der Reaktanten in Wasser 15 bis 70 Masse% beträgt, und das Reaktionsgemisch 10 bis 90 min auf 50 bis 90°C erwärmt und die resultierenden Hydroxyalkyl-modifizierten Dialkenyl-substituierten Imidotriazinderivate durch Sprühtrocknung getrocknet werden, oder die Hydroxyalkyl-modifizierten Dialkenyl-substituierten Imidotriazinderivate durch Zusatz eines Überschusses an C₁-C₃-Alkoholen zur wässrigen Lösung bzw. Dispersion im sauren Medium bei 5 bis 80°C verethert und gegebenenfalls durch azeotrope Umetherung mit C₄-C₁₆-Alkoholen in die entsprechenden C₄-C₁₆-Alkyl-ether übergeführt werden.

7. Verfahren zur Herstellung von Imidotriazinderivaten nach Anspruch 5 oder 6, **dadurch gekennzeichnet, dass** in der ersten Verfahrensstufe als ungesättigtes Dicarbonsäure-anhydrid Maleinsäureanhydrid eingesetzt wird.

8. Verfahren zur Herstellung von Imidotriazinderivaten nach einem oder mehreren der Ansprüche 5 bis 7, **dadurch gekennzeichnet, dass** in der zweiten Verfahrensstufe als Aminotriazinderivat Melamin eingesetzt wird.

9. Verwendung von Imidotriazinderivaten nach einem oder mehreren der Ansprüche 1 bis 4 zur Herstellung von Polymeren, Pharmaka, Biociden, Additiven, Textilhilfsmitteln und Farbstoffen.
